Europäisches Patentamt

European Patent Office

Office européen des brevets

(19)

(11) Veröffentlichungsnummer : **0 059 251**
**B1**

(12) **EUROPÄISCHE PATENTSCHRIFT**

(45) Veröffentlichungstag der Patentschrift :
26.09.84

(21) Anmeldenummer : 81109072.9

(22) Anmeldetag : 28.10.81

(51) Int. Cl.³ : **C 07 C 17/42, C 07 C 21/10, C 09 K 15/04**

(54) **Mittel zur Stabilisierung von Trichlorethylen, sowie das mit diesem Mittel stabilisierte Trichlorethylen.**

(30) Priorität : 04.03.81 DE 3108168

(43) Veröffentlichungstag der Anmeldung :
08.09.82 Patentblatt 82/36

(45) Bekanntmachung des Hinweises auf die Patenterteilung : 26.09.84 Patentblatt 84/39

(84) Benannte Vertragsstaaten :
**BE DE FR GB IT**

(56) Entgegenhaltungen :
**AT-B- 331 769
DE-A- 2 449 667
DE-C- 1 098 506
GB-A- 932 138**

(73) Patentinhaber : **WACKER-CHEMIE GMBH
Prinzregentenstrasse 22
D-8000 München 22 (DE)**

(72) Erfinder : **Blum, Klaus, Dr., Dipl.-Chem.
Hermann-Hiller-Strasse 47
D-8263 Burghausen (DE)**
Erfinder : **Mack, Wilhelm, Dr., Dipl.-Chem.
Unghausen Nr. 24
D-8263 Burghausen (DE)**
Erfinder : **Strasser, Rudolf, Dr., Dipl.-Chem.
Lindacher Strasse 58
D-8263 Burghausen (DE)**

# 0 059 251

### Beschreibung

Es ist hinlänglich bekannt, daß Trichlorethylen praktisch nur in stabilisiertem Zustand handhabbar ist. Dabei muß sich die Stabilisierung insbesondere gegen die zersetzenden Einflüsse von Wärme, Licht, Luft, Feuchtigkeit und Metallen richten.

Gemäß DE-AS 19 06 758 wurde bereits stabilisiertes Trichlorethylen bekannt, das, vorzugsweise zusammen mit Dimethylaminopropin, Cycloheptatrien als Stabilisierungsmittel enthält.

Nach DE-AS 12 00 800 wird zur Stabilisierung von Trichlorethylen eine Mischung von Tetrahydrofuran und N-Methylpyrrol empfohlen.

Weiterhin ist gemäß US-PS 2 371 645 die stabilisierende Wirkung von Ethern, wie Diethylether, Diphenylether und anderen, sowie von als « innere Ether » bezeichneten Epoxiden gegenüber Trichlorethylen bekannt.

Ferner ist aus DE-OS 10 98 506 ein Stabilisierungsmittel für Trichlorethylen bekannt, das neben einer Auswahl von Epoxiden bevorzugt N-Methylpyrrol und Diisobutylen enthält.

Daneben ist gemäß DE-OS 24 49 667 epoxidfrei stabilisiertes Perchlorethylen bekanntgeworden. Als Stabilisierungsmittel wird eine Kombination von N-Alkylmorpholinen, Alkylphenolen und gegebenenfalls Cycloheptatrien oder Cyclooctatetraen eingesetzt. Es werden im beschleunigten Oxidationstest in Gegenwart von Stahl Standzeiten bis zu ca. 400 Stunden erreicht. Die Anforderungen an ein Stabilisierungsmittel für Trichlorethylen sind jedoch wesentlich höher zu setzen, da Trichlorethylen chemisch weniger stabil ist als Perchlorethylen und in großem Umfange zur Leichtmetallentfettung eingesetzt wird. Es sollten daher im beschleunigten Oxidationstest in Gegenwart von Leichtmetall Standzeiten in der Größenordnung von 1 000 Stunden erzielt werden, um den technischen Anforderungen gerecht zu werden.

Den gestiegenen Anforderungen entsprechend haben sich in der Praxis die epoxidhaltigen Stabilisierungsmittel durchgesetzt. Nachteilig an diesen Mitteln ist die Toxizität der Epoxide.

Aufgabe der Erfindung war es, ein epoxidfreies, epoxidhaltigen Mitteln zumindest ebenbürtiges, Stabilisierungsmittel für Trichlorethylen vorzustellen.

Es wurde nun gefunden, daß eine epoxidfreie Stabilisatormischung auf der Grundlage zumindest eines Amins, dessen Siedepunkt zwischen 50 und 150 °C liegt, Ethylacetats, N-Methylpyrrols und/oder eines Alkylphenols, das als weitere Bestandteile Diisobutylen und/oder Cycloheptatrien sowie zumindest eine Verbindung aus der Gruppe der hydroxylgruppenfreien Ether enthält, die obengenannte Aufgabe erfüllt.

Überraschenderweise zeigt das genannte Olefin-Ether-System in Kombination mit der genannten Grundstabilisierung eine synergistisch gesteigerte stabilisierende Wirkung gegenüber Trichlorethylen.

Gegenstand der Erfindung ist ein Mittel zur Stabilisierung von Trichlorethylen auf Basis von zumindest einem Amin, dessen Siedepunkt bei 1 bar zwischen 50 und 150 °C liegt. Ethylacetat, N-Methylpyrrol und/oder zumindest einem Alkylphenol, das dadurch gekennzeichnet ist, daß es als weitere Bestandteile Diisobutylen und/oder Cycloheptatrien und zumindest eine Verbindung aus der Gruppe der hydroxylgruppenfreien Ether enthält.

Bevorzugt werden als hydroxylgruppenfreie Ether solche mit 4 bis 14 Kohlenstoffatomen und 1 bis 4 Ethersauerstoffatomen eingesetzt.

Da es sich erfindungsgemäß um epoxidfreie Stabilisatormischungen handelt, sind die zuweilen als « innere Ether » bezeichneten Epoxide ausgeschlossen.

Erfindungsgemäß können jedoch Cycloalkylether mit zumindest 5 Ringatomen, vorzugsweise 5 bis 6 Ringatomen, jeweils einschließlich Ethersauerstoffatom, eingesetzt werden, wie beispielsweise Tetrahydrofuran, Tetrahydropyran, 1.4-Dioxan, 1.3-Dioxan und andere.

Weitere Beispiele für erfindungsgemäß einzusetzende Ether sind die

Dialkylether, wie Dibutylether, Di-sek.-butylether,

Dialkoxymethane, wie Dimethoxymethan, Diethoxymethan,

Glykoldialkylether, wie Dimethoxyethan, Diethoxyethan, Dipropoxyethan, Butyl-glykol-tert.-butylether,

Polyglykolether, wie Ethyl-diglykol-tert.-butylether, Methoxy-diglykol-tert.-butylether, Triglykoldimethylether und andere,

Arylether, wie Diphenylether,

Aralkylether, wie Dibenzylether,

Arylalkylether, wie Anisol, Hydrochinondimethylether und andere.

Beispiele für erfindungsgemäß einzusetzende Amine, die im Temperaturbereich von 50 bis 150 °C bei 1 bar sieden, sind Triethylamin, Diisopropylamin, Dimethylisobutylamin, sek.-Butylamin, Pentylamin, iso-Pentylamin, 5-Methyl-2-Hexan-amin, Di-isobutylamin, vorzugsweise Diisopropylamin.

Im Rahmen der Erfindung werden unter Alkylphenolen solche Phenole verstanden, die mit 1 bis 3, ggf. verzweigten Alkylketten mit 1 bis 6 Kohlenstoffatomen in o- und/oder p-Stellung substituiert sind. Beispiele sind p-Kresol, o-Kresol, 2.6-Dimethylphenol, 2.4.6-Trimethylphenol, p-Isopropylphenol, p-tert.-Butylphenol, 2.6-Di-tert.-butylphenol, p-Amylphenol und andere.

N-Methylpyrrol kann ganz oder teilweise gegen Alkylphenol ausgetauscht sein.

Weiterhin ist Gegenstand der Erfindung Trichlorethylen, das die erfindungsgemäße Stabilisatormischung enthält. Die Stabilisatormenge liegt in der Regel zwischen 2 000 und 25 000 Gew. ppm, bevorzugt 5 000 bis 10 000 Gew. ppm, jeweils bezogen auf die Gesamtmenge stabilisierten Trichlorethylens.

Vorzugsweise enthält das erfindungsgemäße Trichlorethylen

a) 10 bis 30 Gew. ppm zumindest eines Amins, dessen Siedepunkt bei 1 bar zwischen 50 und 150 °C liegt,

b) 0 bis 400 Gew. ppm N-Methylpyrrol

c) 0 bis 1 000 Gew. ppm Alkylphenol,

mit der Maßgabe, daß die Komponenten gemäß b) und c) zumindest in einer Menge von 20 Gew. ppm vorlangen,

d) 2 000 bis 4 000 Gew. ppm Ethylacetat,

e) 100 bis 10 000 Gew. ppm Diisobutylen und/oder Cycloheptatrien und

f) 100 bis 10 000 Gew. ppm zumindest einer Verbindung aus der Gruppe der hydroxylgruppenfreien Ether.

Die Komponenten a), b), c) und d) sind als Grundstabilisierung zu betrachten, auf deren Basis sich durch Zusatz des erfingungsgemäßen Olefin/Ether-Systems eine synergistisch gesteigerte Stabilisatorwirkung entfaltet.

Zur Prüfung auf Stabilität wurde das erfindungsgemäße Trichlorethylen den beiden folgenden Tests unterworfen :

1. Es wurde der beschleunigte Oxidationstest nach MIL-T-81533A durchgeführt. Als Testapparatur diente ein mit 200 ml erfindungsgemäß stabilisierten Trichlorethylens gefülltes Siedegefäß mit Rückflußkühler, das mittels einer 150 Watt Glühlampe beheizt wurde. In die Testflüssigkeit wurde ein stetiger wasserdampfgesättigter Sauerstoffstrom (10 bis 12 Blasen pro Minute) geleitet. Weiter war ein Streifen aus metallischem Aluminium so angeordnet, daß er zur Hälfte in die Testflüssigkeit eintauchte. Ein zweiter Aluminiumstreifen befand sich am Gefäßboden.

Als Stabilitätskriterium wurde die Zeit herangezogen, die das stabilisierte Trichlorethylen den obigen Testbedingungen ausgesetzt werden mußte, bis ein HCl-Gehalt von 0,02 Gew.% erreicht war. Der HCl-Gehalt wurde durch regelmäßige Probeentnahme und anschließende Titration mit 0,1 nNaOH gegen Phenolphthalein ermittelt.

2. Als weitere Qualitätsprüfung diente eine von der Bundesanstalt für Materialprüfung vorgeschlagene Test-methode (BAM-Test) : hierzu wurden 100 ml erfindungsgemäß stabilisierteń Trichlorethylens mit 100 ml Toluol vermischt und zusammen mit 18 g Aluminiumflitter einer Teilchengröße < 0,5 mm und zusammen mit 0,7 g wasserfreien Aluminiumchlorids 18 Stunden am Rückfluß gekocht. Eine zweite, im übrigen gleiche Probe enthielt zusätzlich 1 g Zinkstearat und eine dritte Probe zusätzlich 10 ml Ölsäure. Schließlich wurden die drei beschriebenen Tests noch mit Destillaten des erfindungsgemäß stabilisierten Trichlorethylens wiederholt.

Das Prüfverfahren galt als bestanden, wenn bei keinem der beschriebenen Einzeltests exotherme Reaktionen auftraten.

Die Erfindung wird nun anhand von Beispielen und Vergleichsbeispielen näher erläutert. Die Daten beziehen sich auf die Ergebnisse der beiden oben beschriebenen Tests. Das Zeichen (−) steht für keine Reaktion während des BAM-Tests.

Beispiel 1

3 kg Trichlorethylen wurden mit 0,08 g Diisopropylamin, 0,9 g N-Methylpyrrol und 8,4 g Ethylacetat als Grundstabilisierung versetzt. Dem grundstabilisierten Trichlorethylen wurden weiter 12,6 g Dimethoxyethan und 8,4 g Diisobutylen zugesetzt.

Der BAM-Test verlief ohne Reaktion. Der beschleunigte Oxidationstest nach MIL-T-81533A wurde nach 1 340 Stunden abgebrochen. Es wurde ein HCl-Gehalt von lediglich 0,005 Gew.% ermittelt.

Vergleichsbeispiel 1

Es wurde stabilisiertes Trichlorethylen analog Beispiel 1 — jedoch ohne den Zusatz von Diisobutylen — untersucht.

Im Oxidationstest erreichte die Probe nach 192 Stunden die Aciditätsgrenze von 0,02 Gew.% HCl.

Vergleichsbeispiel 2

Es wurde stabilisiertes Trichlorethylen analog Beispiel 1 — jedoch ohne den Zusatz von Dimethoxyethan — untersucht. Im Oxidationstest erreichte die Probe nach 694 Stunden die Aciditätsgrenze von 0,02 Gew.% HCl.

3

Vergleichsbeispiel 3

Es wurde Trichlorethylen mit einem Gehalt an

      20 Gew. ppm Diisopropylamin
   300 Gew. ppm N-Methylpyrrol
2 800 Gew. ppm Ethylacetat und
7 000 Gew. ppm Diisobutylen

untersucht. Die Aciditätsgrenze von 0,02 Gew.% HCl war nach 900 Stunden erreicht.

Vergleichsbeispiel 4

Es wurde Trichlorethylen mit einem Gehalt an

      20 Gew. ppm Diisopropylamin
   300 Gew. ppm N-Methylpyrrol
2 800 Gew. ppm Ethylacetat
2 800 Gew. ppm Diisobutylen
4 200 Gew. ppm Glykol-mono-methylether

getestet. Die Aciditätsgrenze von 0,02 Gew.% HCl war nach 189 Stunden erreicht.

Weitere Ergebnisse von Beispielen und Vergleichsbeispielen werden in der folgenden Tabelle aufgeführt :

(Siehe Tabelle Seite 5 ff.)

4

Tabelle

| Beispiel | Vergleichs-beispiel | Gew.ppm Stabilisator | Oxidationstest nach MIL-T-81533A Stunden nach Erreichen der Aciditätsgrenze von 0,02 Gew.% | BAM-Test |
|---|---|---|---|---|
| 2 | | 20 Diisopropylamin<br>300 N-Methylpyrrol<br>2800 Ethylacetat<br>2800 Diisobutylen<br>4200 Di-sek.-butylether | ⟩ 1410 | - |
| 3 | | 20 Diisopropylamin<br>300 N-Methylpyrrol·<br>2800 Ethylacetat<br>2800 Diisobutylen<br>4200 Butylglykol-tert.-butylether | 1415 | - |
| 4 | | 20 Diisopropylamin<br>300 N-Methylpyrrol<br>2800 Ethylacetat<br>2800 Diisobutylen<br>4200 Ethyldiglykol-tert.-butylether | ⟩ 1410 | - |
| 5 | | 20 Diisopropylamin<br>300 N-Methylpyrrol<br>2800 Ethylacetat<br>2800 Diisobutylen<br>4200 Triglykoldimethyl-ether | 1200 | - |

(Fortsetzung)

| Beispiel | Vergleichs-beispiel | Gew.ppm Stabilisator | Oxidationstest nach MIL-T-81533A Stunden nach Erreichen der Aciditätsgrenze von 0,02 Gew.% | BAM-Test |
|---|---|---|---|---|
| 6 | | 20 Diisopropylamin<br>300 N-Methylpyrrol<br>2800 Ethylacetat<br>2800 Diisobutylen<br>4200 1.4-Dioxan | 1123 | - |
| | 5 | 20 Diisopropylamin<br>300 N-Methylpyrrol<br>2800 Ethylacetat<br>4200 1.4-Dioxan | 669 | - |
| 7 | | 20 Diisopropylamin<br>300 N-Methylpyrrol<br>2800 Ethylacetat<br>2800 Diisobutylen<br>4200 Tetrahydrofuran | 1220 | - |
| | 6 | 20 Diisopropylamin<br>300 N-Methylpyrrol<br>2800 Ethylacetat<br>4200 Tetrahydrofuran | 548 | - |
| 8 | | 20 Diisopropylamin<br>300 N-Methylpyrrol<br>2800 Ethylacetat<br>2800 Diisobutylen<br>4200 Diphenylether | 1300 | - |
| 9 | | 20 Diisopropylamin<br>300 N-Methylpyrrol<br>2800 Ethylacetat<br>2800 Diisobutylen<br>4200 Hydrochinondimethyl-ether | >1650 | - |

0 059 251

(Fortsetzung)

| Beispiel | Vergleichs-beispiel | Gew.ppm Stabilisator | Oxidationstest nach MIL-T-81533A Stunden nach Erreichen der Aciditätsgrenze von 0,02 Gew.% | BAM-Test |
|---|---|---|---|---|
| 10 | | 20 Diisopropylamin<br>300 N-Methylpyrrol<br>2800 Ethylacetat<br>2800 Diisobutylen<br>4200 Dibenzylether | > 1818 | - |
| | 7 | 20 Diisopropylamin<br>300 N-Methylpyrrol<br>2800 Ethylacetat<br>4200 Dibenzylether | 840 | (-) |
| 11 | | 20 Diisopropylamin<br>300 N-Methylpyrrol<br>2800 Ethylacetat<br>2800 Diisobutylen<br>4200 Anisol | 1745 | - |
| | 8 | 20 Diisopropylamin<br>300 N-Methylpyrrol<br>2800 Ethylacetat<br>4200 Anisol | 333 | (-) |
| 12 | | 20 Diisopropylamin<br>300 N-Methylpyrrol<br>2800 Ethylacetat<br>2800 Cycloheptatrien<br>4200 Anisol | > 1415 | - |
| 13 | | 20 Diisopropylamin<br>300 N-Methylpyrrol<br>2800 Ethylacetat<br>2800 Cycloheptatrien<br>4200 Ethyldiglykol-tert.-Butylether | 1200 | - |

(Fortsetzung)

| Beispiel | Vergleichs-beispiel | Gew.ppm Stabilisator | Oxidationstest nach MIL-T-81533A Stunden nach Erreichen der Aciditätsgrenze von 0,02 Gew.% | BAM-Test |
|---|---|---|---|---|
| 14 | | 20 Diisopropylamin<br>553 2.6-Dimethylphenol<br>2800 Ethylacetat<br>2800 Diisobutylen<br>4200 Hydrochinondimethyl-ether | 1410 | - |
| 15 | | 20 Diisopropylamin<br>553 2.6-Dimethylphenol<br>2800 Ethylacetat<br>2800 Diisobutylen<br>4200 Dibenzylether | 1350 | - |

## Ansprüche

1. Mittel zur Stabiliserung von Trichlorethylen auf Basis von zumindest einem Amin, dessen Siedepunkt bei 1 bar zwischen 50 und 150 °C liegt, Ethylacetat, N-Methylpyrrol und/oder zumindest einem Alkylphenol, dadurch gekennzeichnet, daß es als weitere Bestandteile einerseits Diisobutylen und/oder Cycloheptatrien und andererseits zumindest eine Verbindung aus der Gruppe der hydroxylgruppenfreien Ether enthält.

2. Mittel nach Anspruch 1, dadurch gekennzeichnet, daß als Ether solche mit 4 bis 14 Kohlenstoffatomen und 1 bis 4 Ethersauerstoffatomen eingesetzt werden.

3. Stabilisiertes Trichlorethylen, dadurch gekennzeichnet, daß es ein Mittel nach mindestens einem der Ansprüche 1 bis 2 enthält.

4. Trichlorethylen nach Anspruch 3, dadurch gekennzeichnet, daß es 2 000 bis 25 000 Gew. ppm Stabilisierungsmittel enthält.

5. Trichlorethylen nach Anspruch 3, enthaltend

a) 10 bis    30 Gew. ppm zumindest eines Amins, dessen Siedepunkt bei 1 bar zwischen 50 und 150 °C liegt,
b)  0 bis   400 Gew. ppm N-Methylpyrrol
c)  0 bis 1 000 Gew. ppm Alkylphenol

mit der Maßgabe, daß die Komponenten gemäß b) und c) zumindest in einer Menge von 20 Gew. ppm vorliegen,

d) 2 000 bis  4 000 Gew. ppm Ethylacetat
e)    100 bis 10 000 Gew. ppm Diisobutylen und/oder Cycloheptatrien und
f)    100 bis 10 000 Gew. ppm zumindest einer Verbindung aus der Gruppe der hydroxylgruppenfreien Ether.

## Claims

1. Agent for stabilising trichloroethylene, based on at least one amine having a boiling point of from 50 to 150 °C at 1 bar, ethyl acetate, and N-methylpyrrole and/or at least one alkylphenol, characterised in that it contains as further constituents, first diisobutylene and/or cycloheptatriene, and secondly at least one compound selected from the group consisting of ethers that contain no hydroxyl groups.

2. Agent according to claim 1, characterised in that, as ethers, those having from 4 to 14 carbon atoms and from 1 to 4 ether oxygen atoms are used.

3. Stabilised trichloroethylene, characterised in that it contains an agent according to at least one of claims 1 and 2.

4. Trichloroethylene according to claim 3, characterised in that it contains from 2,000 to 25,000 ppm by weight of the stabilising agent.

5. Trichloroethylene according to claim 3, containing

a) from 10 to    30 ppm by weight of at least one amine having a boiling point of from 50 to 150 °C at 1 bar,
b) from  0 to   400 ppm by weight of N-methylpyrrole,
c) from  0 to 1,000 ppm by weight of alkylphenol,

with the proviso that at least 20 ppm by weight of the components according to b) and c) are present,

d) from 2,000 to  4,000 ppm by weight of ethyl acetate,
e) from    100 to 10,000 ppm by weight of diisobutylene and/or cycloheptatriene and
f) from    100 to 10,000 ppm by weight of at least one compound selected from the group consisting of ethers that contain no hydroxyl groups.

## Revendications

1. Produit pour stabiliser le trichloréthylène, à base d'au moins une amine dont le point d'ébullition sous 1 bar est compris entre 50 et 150 °C, d'acétate d'éthyle, de N-méthyl-pyrrole et/ou d'au moins un alkylphénol, produit caractérisé en ce qu'il contient, comme constituants supplémentaires, d'une part du di-isobutylène et/ou du cycloheptatriène et, d'autre part, au moins un composé pris dans l'ensemble des éthers dépourvus de radical hydroxy.

2. Produit selon la revendication 1 caractérisé en ce qu'il contient, comme éthers, des éthers renfermant de 4 à 14 atomes de carbone et de 1 à 4 atomes d'oxygène de fonction éther.

3. Trichloréthylène stabilisé caractérisé en ce qu'il contient un produit selon l'une des revendications 1 et 2.

4. Trichloréthylène selon la revendication 3 caractérisé en ce qu'il contient de 2 000 à 25 000 ppm en poids du produit stabilisant.

5. Trichloréthylène selon la revendication 3 qui contient :

a) de 10 à 30 ppm en poids d'au moins une amine dont le point d'ébullition sous 1 bar est compris entre 50 et 150 °C,

b) de 0 à 400 ppm en poids de N-méthyl-pyrrole,

c) de 0 à 1 000 ppm en poids d'un alkylphénol,

avec la condition que les composantes (b) et (c) soient présentes en une quantité au moins égale à 20 ppm en poids,

d) de 2 000 à 4 000 ppm en poids d'acétate d'éthyle,

e) de 100 à 10 000 ppm en poids de di-isobutylène et/ou de cycloheptatriène, et

f) de 100 à 10 000 ppm en poids d'au moins un composé pris dans l'ensemble des éthers dépourvus de radical hydroxy.